# FASCICULE DE BREVET EUROPEEN

(11) **EP 2 240 479 B1**
(45) Date de publication et mention de la délivrance du brevet: **20.04.2011**
(21) Numéro de dépôt: 08872501.5
(22) Date de dépôt: 10.12.2008
(51) Int. Cl.: C07D 471/04, C08F 4/00

(54) **NOUVEAUX COMPOSES ORGANIQUES AZOTÉS UTILISABLES COMME PRÉCURSEUR DE COMPOSITION CATALYTIQUE**
NEUARTIGE ALS VORLÄUFER EINER KATALYSATORZUSAMMENSETZUNG VERWENDBARE STICKSTOFFHALTIGE ORGANISCHE VERBINDUNGEN
NOVEL NITROGEN-CONTAINING ORGANIC COMPOUNDS THAT CAN BE USED AS PRECURSOR OF A CATALYTIC COMPOSITION

(30) Priorité: 04.01.2008 FR 0800061
(43) Date de publication de la demande: 20.10.2010
(73) Titulaire: IFP Energies nouvelles, 92852 Rueil-Malmaison Cedex (FR)
(72) Inventeur: RANGHEARD, Claudine, 69006 Lyon (FR); OLIVIER-BOURBIGOU, Hélène, F-69230 Saint Genis Laval (FR); PRORIOL, David, F-69530 Brignais (FR)
(86) Numéro de dépôt international: PCT/FR2008/001720
(87) Numéro de publication internationale: WO 2009/103879

(56) Documents cités:
- EP-A- 0 816 385
- WO-A-03/011876
- US-B1- 6 730 788
- JIE ET AL: "Iron(II) complexes ligated by 2-imino-1,10-phenanthrolines: Preparation and catalytic behavior toward ethylene oligomerization" JOURNAL OF MOLECULAR CATALYSIS. A, CHEMICAL, ELSEVIER, AMSTERDAM, NL, vol. 269, no. 1-2, 3 avril 2007 (2007-04-03), pages 85-96, XP022015761 ISSN: 1381-1169
- RANGHEARD ET AL: "Direct synthesis of a new class of N,N,N ligands based on 1,2-dihydro-1,10-phenanthroline backbone and their coordination to Pd complexes" J. CHEM. SOC. DALTON TRANS. 2009,, no. 5, 12 décembre 2008 (2008-12-12), pages 770-772, XP002529553

## Description

### DOMAINE DE L'INVENTION

La présente invention concerne de nouveaux composés organiques azotés ayant des applications comme ligand de métaux de transition. Lesdits composés peuvent être utilisés pour la catalyse en particulier pour la dimérisation, la codimérisation, l'oligomérisation ou la polymérisation des oléfines.

### ART ANTÉRIEUR

Les α-oléfines linéaires, spécialement celles qui contiennent de 4 à 20 atomes de carbone, possédant des débouchés variés en fonction de la longueur de leur chaîne carbonée. Par exemple, les oléfines C4 à C8 sont principalement utilisées comme co-monomères pour la fabrication de polyéthylènes basse densité (LLDPE), les C8 à C14 comme intermédiaires dans l'industrie des lubrifiants et les C8-C18 pour la fabrication des détergents. Ces oléfines connaissent une forte croissance économique. La plupart des procédés industriels de production d'α-oléfines sont des procédés d'oligomérisation de l'éthylène catalysés par des complexes des métaux de transition.

On assiste donc depuis plusieurs années à une recherche incessante pour trouver de nouveaux ligands dans le domaine de la catalyse homogène pouvant conduire à des sytèmes plus sélectifs, plus actifs et éventuellement recyclables, et ceci notamment pour la polymérisation ou l'oligomérisation de l'éthylène. Les ligands azotés de type bis-imino-pyridines ou imino-pyridines ont suscité un intérêt particulier, en particulier pour leurs propriétés stériques et électroniques modulables et pour leur coordination de façon tridente ou bidente sur les métaux de transition. L'article publié récemment (Chem. Rev. 2007, 107, 1745-1776) présente les derniers développements réalisés autour de cette famille de ligands.

De façon surprenante, nous avons découvert de nouveaux composés organiques azotés pouvant être utilisés dans le domaine de la catalyse homogène.

### RÉSUMÉ DE L'INVENTION

Des derivés de phenantroline utilisables dans la préparation de catalyseurs sont décrit dans Journal of Molecular Catalysis, 269, 2007, 85 EP 816 385 et US 6730788.

La présente invention concerne de nouveaux composés organiques azotés présentant un fort potentiel pour des applications en catalyse homogène, obtenus en faisant réagir un composé X de type pyridine substituée avec un composé Y de type amino-quinoléine.

### DESCRIPTION DÉTAILLÉE DE L'INVENTION

La présente invention décrit le composé organique azoté A utilisable comme ligand en catalyse homogène ayant la formule générale suivante : 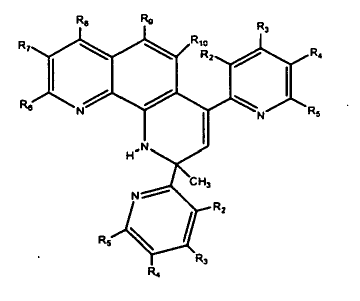 dans laquelle R₂ à R₁₀, identiques ou différents, sont choisis parmi l'hydrogène, des groupements alkyles, saturés ou non saturés, cycloalkyles ou aromatiques, aryles ou aralkyles, éventuellement substitués, des groupements alcoxy, aryloxy ou amino, un halogénure.

Dans le composé A, les groupements R₂ à R₁₀, identiques ou différents, peuvent également représenter des radicaux organiques dans lesquels un ou plusieurs atomes d'hydrogène sont remplacés par des halogénures, par exemple un fluorure, ou des groupements comportant au moins un hétéroatome tel que un oxygène, un azote, un soufre, un phosphore ou un silicium. Ces hétéroéléments peuvent être contenus dans des cycles alkyles saturés ou insaturés ou aromatiques.

La présente invention décrit le composé organique azoté B utilisable comme ligand en catalyse homogène ayant la formule générale suivante : 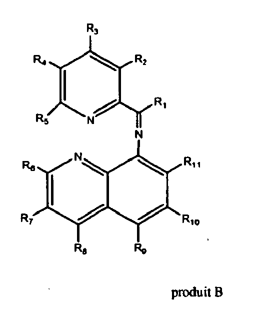 dans laquelle R₁ à R₁₁, identiques ou différents, sont choisis parmi l'hydrogène, des groupements alkyles, saturés ou non saturés, cycloalkyles ou aromatiques, aryles ou aralkyles, éventuellement substitués, des groupements alcoxy, aryloxy ou amino, un halogénure, R₁ étant différent du radical méthyle.

Dans le composé B, les groupements R₁ à R₁₁, identiques ou différents, peuvent également représenter des radicaux organiques dans lesquels un ou plusieurs atomes d'hydrogène sont remplacés par des halogénures, par exemple un fluorure, ou des groupements comportant au moins un hétéroatome tel que un oxygène, un azote, un soufre, un phosphore ou un silicium. Ces hétéroéléments peuvent être contenus dans des cycles alkyles saturés ou insaturés ou aromatiques.

La présente invention décrit également le procédé de fabrication des produits A et B comprenant au moins une étape dans laquelle on fait réagir, de préférence dans un solvant, un composé X appartenant à la famille des pyridines substituées comportant au moins une fonction cétone, avec un composé Y appartenant à la famille des amino-quinoléines et de leurs dérivés.

Le procédé selon la présente invention peut également comprendre au moins une étape supplémentaire permettant de réaliser une réaction de substitution de groupements.

Le composé X répond à la formule générale suivante :

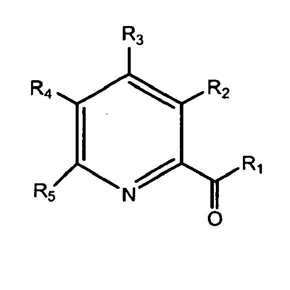

Le composé Y appartient à la famille des amino-quinoléines et de leurs dérivés. La formule générale correspondante est écrite ci-après : 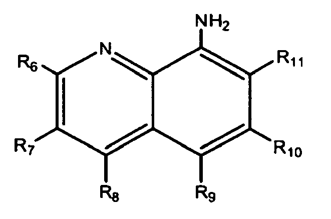 avec R₁ à R₁₁, identiques ou différents, sont choisis parmi l'hydrogène, des groupements alkyles, saturés ou non saturés, cycloalkyles ou aromatiques, aryles ou aralkyles, éventuellement substitués, des groupements alcoxy, aryloxy ou amino, ou les halogénures, R₁ étant différent de l'hydrogène.

Le composé X est par exemple la 2-acétylpyridine, 4-méthyl-2-acétylpyridine, 2-bromo-6-acétylpyridine, 6-méthyl-2-acétylpyridine, 2-méthoxy-6-acétylpyridine.

Le composé Y est par exemple la 8-aminoquinoléine, ou le 2-méthyl-8-aminoquinoléine.

La réaction entre les deux composés X et Y est réalisée de préférence dans un solvant, à une température comprise de préférence entre 20 et 250°C. Les composés X et Y peuvent être introduits dans un ordre quelconque.

Les solvants utilisés sont choisis parmi les solvants organiques classiques polaires ou apolaires, protiques ou aprotiques, tels que les hydrocarbures aromatiques ou aliphatiques tels que le toluène, le xylène, le cyclohexane, les solvants chlorés tels que le dichorométhane, les solvants nitrés tel que l'acétonitrile, les alcools tels que le méthanol ou l'éthanol. Ces solvants peuvent être utilisés seuls ou en mélange. Ces solvants sont de préférence séchés, par distillation ou par passage sur un adsorbant, avant d'être utilisés.

La réaction de X avec Y est de préférence réalisée en présence d'un catalyseur. Les catalyseurs sont de préférence choisis parmi les acides de Bronsted ou les acides de Lewis.

Les acides de Bronsted sont de type H⁺X⁻ dans laquelle X⁻ représente un anion. Les anions X⁻ sont de préférence choisis parmi les anions tétrafluoroborate, tétraalkylborates, hexafluorophosphates, hexafluoroantimonates, alkylsulfonates (par exemple le méthylsulfonate), p-toluènesulfonates, perfluorosulfonates (par exemple le trifluorométhylsulfonate), fluorosulfonates, sulfates, phosphates, perfluoroacétates (par exemple le trifluoroacétate), perfluorosulfonamides (par exemple l'amidure de bis-trifluorométhanesulfonyle de formule N(CF₃SO₂)₂⁻), fluorosulfonamides, perfluorosulfométhides (par exemple le méthylure de tris-trifluorométhanesulfonyle de formule C(CF₃SO₂)₃⁻), carboranes, tétraphénylborates et les anions tétraphénylborates dont les noyaux aromatiques sont substitués.

Les acides de Lewis sont par définition des composés susceptibles d'accepter un doublet d'électrons.

A titre d'exemple, on peut citer les triflates de lanthanides, en particulier le triflate d'ytterbium (Yb(OTf)₃), le triflate de scandium.

La réaction entre X et Y peut éventuellement être réalisée en présence d'iode.

La réaction entre les composés X et Y libère de l'eau. L'eau peut être avantageusement piégée durant la réaction en ajoutant un desséchant tel que du tamis moléculaire. Elle peut être également éliminée par distillation azéotropique avec le solvant de la réaction.

Le rapport molaire entre le composé X et le composé Y est compris entre 10 et 0,1, de préférence entre 5 et 0,2.

Le produit principal obtenu lors de la réaction de X avec Y peut être isolé et purifié selon les méthodes classiques utilisées en chimie organique telles que la précipitation, la cristallisation ou la séparation par chromatographie en phase liquide sur une colonne d'alumine ou de silice.

Les exemples suivants illustrent l'invention sans en limiter la portée.

### EXEMPLES

### Exemple 1 :

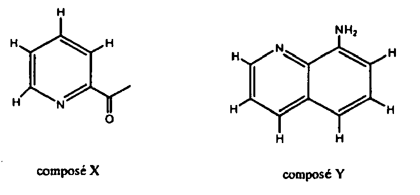

La condensation de 6,89 g de 2-acétylpyridine (56,9 mmol) et de 4,1 g de 8-aminoquinoléine (28,4 mmol), avec 1,3 mL d'acide formique HCOOH est réalisée dans 75 mL de MeOH anhydre. Le milieu réactionnel est agité à reflux pendant 72h. Après évaporation du méthanol sous vide, la cétone est évaporée en tirant sous vide et en chauffant à 60°C. Le produit brut obtenu est purifié par chromatographie sur colonne d'alumine pour éliminer la 8-amino quinoléine, puis sur silice neutre (éluant CH₂Cl₂/AcOEt 80/20). 2,4 g d'un solide jaunâtre sont obtenus. Le rendement obtenu est de 30%.

Les caractérisations sont faites par des méthodes d'analyse de RMN du ¹H et du ¹³C, par spectroscopie IR et par spectroscopie de masse GC/MS.

RMN ¹H : δ_{H} (300 MHz, CD₂Cl₂) 1.90 (s, 3H), 6.17 (d, 1H, *J* 2.3 Hz), 6.95 (d, 1H, *J* 8.6 Hz), 7.00 (s, 1H), 7.11 (ddd, 1H, *J* 7.3, 4.7 et 1.4 Hz), 7.29 (ddd, 1H, *J* 7.6, 4.7 et 1.2 Hz), 7.32 (d, 1H, *J* 8.5 Hz), 7.35 (dd, 1H, *J* 8.2 et 4.35Hz), 7.49 (dt, 1H, *J* 7.9 et 1.1 Hz), 7.59 (dt, 1H, *J* 7.94 et 1.15Hz), 7.62 (td, 1H, *J* 7.5 et 1.9 Hz), 7.76 (td, 1H, *J* 7.65 et 1.2Hz), 8.02 (dd, 1H, *J* 8.3 et 1.8 Hz), 8.60 (dq, 1H, *J* 4.8 et 0.9 Hz), 8.69 (dq, 1H, *J* 4.9 et 0.9 Hz), 8.75 (dd, 1H, *J* 4.2 et 1.7 Hz) ppm ;

RMN ¹³C : δ_{C} (75MHz, CD₂Cl₂) 31.1, 59.1, 113.8, 115.5, 120.3, 121.8, 121.9, 122.7, 123.9, 125.1, 128.9, 130.0, 136.1, 136.5, 136.8, 136.9, 137.7, 140.6. 148.0, 149.61, 149.64, 158.2, 166.6 ppm ;

IR : 3371, 3048, 2968, 1632 , 1583, 1563, 1508, 1464, 1428, 1378, 1294, 1225, 1100, 1044, 991, 823, 804, 783, 750 cm⁻¹.

GC/MS : 350, 335, 272, 256, 167.

La formule développée du produit obtenu A est la suivante :

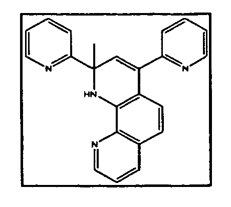

**Exemple 2 :**

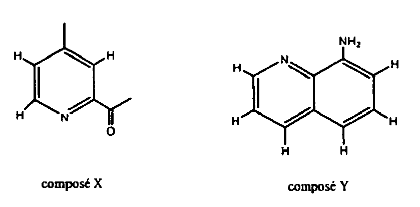

La synthèse est réalisée comme dans l'Exemple 1, à ceci près que l'on utilise 4,00 g de 4-méthyl-2-acétylpyridine (29,6 mmol) et 4,27 g de 8-aminoquinoléine (29,6 mmol).

Ce mélange est mis en solution dans 60 mL de MeOH distillé avec 0,8 mL d'acide formique puis chauffé à reflux pendant 96 heures.

1,8 g d'un solide jaune vif sont obtenus, ce qui correspond à un rendement de 32%.

Le solide est caractérisé par RMN ¹H, ¹³C ,par IR et spectroscopie de masse.

RMN ¹H : δ_{H} (300 MHz, CD₂Cl₂) 1.85 (s, 3H), 2.28 (s, 3H), 2.40 (s, 3H), 6.10 (d, 1H, *J* 2.3 Hz), 6.93 (d, 1H, *J* 8.6 Hz), 6.96 (m, 1H), 7.12 (dm, 1H, *J* 5.0Hz), 7.30-7.40 (m, 4H), 8.01 (dd, 1H, *J* 8.4 et 1.4 Hz), 8.44 (d, 1H, *J* 4.9 Hz), 8.52 (d, 1H, *J* 5.1 Hz), 8.74 (dd, 1H, *J* 4.2 et 1.7 Hz) ppm ;

RMN ¹³C : δ_{C} (75MHz, CD₂Cl₂) 21.2, 21.4, 31.2, 59.1, 113.7. 115.4, 121.2, 121.8, 122.9, 123.7, 124.7, 125.3, 128.9, 129.6, 136.1, 136.4, 137.8, 140.6, 147.9, 148.1, 148.2, 149.31, 149.32, 158.2, 166.4 ppm ;

IR : 3359, 2974, 2921, 2822, 1640 , 1599, 1555, 1509, 1467, 1448, 1423, 1378, 1350, 1116, 1090, 1031, 991, 847, 827, 803, 779, 711, 696 cm⁻¹.

MS : EI *m*/*z* 363, 286, 270, 256, 243, 189, 181.

La formule développée du produit obtenu A est la suivante :

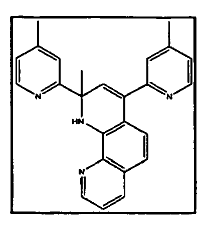

**Exemple 3 :**

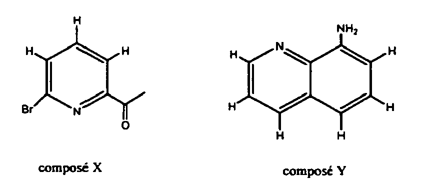

Synthèse de la 2-bromo-6-acétylpyridine (composé X) :

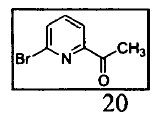

12,5 mL d'une solution de n-BuLi 1,6 M dans l'hexane (20 mmol) sont ajoutés à -78°C à une solution de 4,70 g de 2,6-dibromopyridine (20 mmol) dans 100 mL d'éther anhydre. Le milieu réactionnel est laissé à froid pendant 30 min. 2 mL de N,N-diméthylacétamide sont ajoutés à froid (-78°C) pendant 1h puis laissés remonter à température ambiante. Le milieu réactionnel est neutralisé avec 25 mL d'une solution aqueuse de HCl 1 M. La phase aqueuse est extraite avec Et₂O (3x20 mL). Les phases organiques réunies sont lavées avec de la saumure (3x20 mL), séchées sur MgSO₄, filtrées puis évaporées sous vide. Le produit brut est recristallisé dans un mélange Et₂O /n-C₅H₁₂ (1/2). 1,18 g de solide orange sont obtenus soit un rendement de 28%.

δ_{H} (300 MHz, CD₂Cl₂) 2.66 (s, 3H), 7.37 (dd, 1H, *J* 7.9 et 1.5 Hz), 7.72 (dd, 1H, *J* 7.9 et 7.0 Hz), 7.96 (dd, 1H, *J* 7.0 et 1.5 Hz) ppm.

### Réaction de la 2-bromo-6-acétylpyridine avec la 8-aminoquinoléine :

0,5 g de 2-bromo-6-acetylpyridine (2,5 mmol), 0,36g de 8-aminoquinoline (2,5 mmol) sont dissous dans 4,25 mL de méthanol fraîchement distillé. 0,05mL d'HCOOH sont ajoutés et le milieu réactionnel est porté à reflux pendant 4 jours. Le précipité apparu est filtré puis lavé au MeOH à froid puis séché sous vide. 0,35g (0,68 mmol) de solide jaune pure sont obtenus, ce qui correspond à un rendement de 54%.

RMN ¹H : δ_{H} (300 MHz, CD₂Cl₂) 1.89 (s, 3H), 6.13 (d, 1H, *J* 2.3 Hz), 6.87 (s, 1H), 7.00 (s, 1H, *J* 8.7 Hz), 7.27-7.33 (m, 2H), 7.39 (dd, 1H, *J* 8.4 et 4.2 Hz), 7.43-7.54 (m, 4H), 7.63 (t, 1H, *J* 7.7Hz), 8.05 (dd, 1H, *J* 8.3 et 1.7 Hz), 8.76 (dd, 1H, *J* 4.2 et 1.7 Hz) ppm ;

RMN¹³C : δ_{C} (75MHz, CD₂Cl₂) 30.5, 59.1, 114.5, 114.8, 119.5, 122.1, 122.9, 124.6, 126.4, 127.2, 129.4, 130.2, 135.5, 136.2, 137.7, 139.4, 139.6, 140.3, 141.8, 142.2, 148.2, 159.0, 168.1 ppm;

IR : 3370, 3064, 1635, 1576, 1548, 1508, 1465, 1441, 1417, 1402, 1375, 1363, 1223, 1184, 1164, 1151, 1119, 1090, 1046, 1019, 986, 922, 848, 823, 797, 770, 746, 705, 696, 668 cm⁻¹.

MS : EI *m*/*z* 508, 493, 413, 350, 270.

La formule développée du produit obtenu A est la suivante :

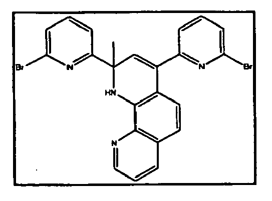

### Exemple 4:

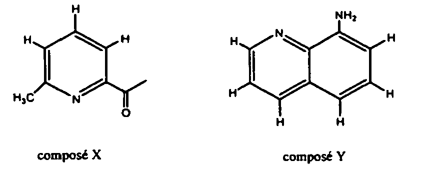

La condensation de 1,95 g de 6-méthyl-2-acétyl-pyridine (14,4 mmol) et de 2,08 g de 8-aminoquinoléine (14,4 mmol), avec 0,4 mL d'acide formique HCOOH est réalisée dans 30 mL de MeOH anhydre. Le milieu réactionnel est agité à reflux pendant 96 h. Après évaporation du méthanol sous vide, le produit brut est purifié sur une colonne de silice (éluant : CH2Cl2/acétate d'éthyle 95/5). 0,8 g d'un solide jaune vif sont obtenus (rendement 17%).

RMN ¹H : δ_{H} (300 MHz, CD₂Cl₂) 1.87 (s, 3H), 2.54 (s, 3H), 2.59 (s, 3H), 6.12 (d, 1H, *J* 2.3 Hz), 6.93 (d,H, *J* 8.7 Hz), 6.94 (s, 1H), 6.97 (dt, 1H, *J* 7.5 et 0.75Hz), 7.15 (dm, 1H, *J* 7.7Hz), 7.27 (dm, 1H, *J* 7.3Hz), 7.29-7.38 (m, 3H), 7.50 (t, 1H, *J* 7.7 Hz), 7.64 (t, 1H, *J* 7.7 Hz), 8.01 (dd, 1H, *J* 8.2 et 1.7 Hz), 8.75 (dd, 1H, *J* 4.2 et 1.8 Hz) ppm ;

RMN ¹³C : δ_{C} (75MHz, CD₂Cl₂) 24.69, 24.72, 31.1, 59.1, 113.7, 115.6, 117.1, 120.9, 121.4, 121.7, 122.1, 125.3, 128.9, 129.9, 136.1, 136.3, 137.1, 137.7, 140.6, 147.9, 157.7, 158.36, 158.49, 165.9 ppm ;

IR : 3373, 3055, 2965, 2920, 1733, 1637, 1586, 1572, 1511, 1478, 1456, 1384, 1256, 1225, 1167, 1124, 1099, 1063, 996, 856, 818, 806, 798, 752, 703 cm⁻¹.

MS : EI *m*/*z* 363, 286, 270, 256, 243, 189, 181.

La formule développée du produit obtenu A est la suivante :

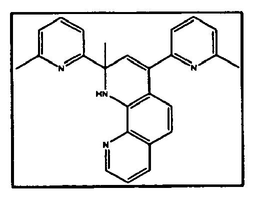

### Exemple 5 :

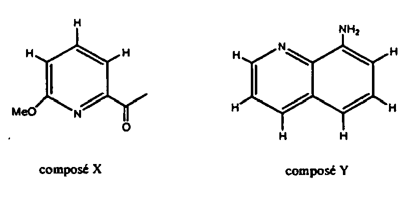

### Synthèse de la 2-methoxy-6-acétylpyridine :

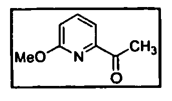

5,1 mL d'une solution de n-BuLi 1,6 M dans l'hexane (8,14 mmol) sont ajoutés à -78°C à une solution de 1 mL (8,16 mmol). Le milieu réactionnel est laissé remonter à température ambiante pendant 30 min. 0,81 mL de N,N-diméthylacétamide sont ajoutés à -78°C et le milieu réactionnel est agité à froid pendant 30 min puis laisser remonter à température ambiante. Le milieu réactionnel est neutralisé avec 1 mL d'une solution aqueuse de HCl 1 M. La phase aqueuse est extraite avec Et₂O (3x10mL). Les phases organiques réunies sont lavées avec de la saumure (3x10 mL), séchées sur MgSO₄, filtrées puis évaporées sous vide.1,03 g (6,08 mmol)de solide beige sont obtenus (rendement : 84%).

δ_{H} (300 MHz, CD₂Cl₂) 2.65 (s, 3H), 3.99 (s, 3H), 6.93 (dd, 1H, *J* 7.3 et 0.9 Hz), 7.58 (dd, 1H, *J* 6.7 et 0.9 Hz), 7.71 (dd, 1H, *J* 8.8 et 7.3 Hz) ppm.

### Réaction de la 2-methoxy-6-acétylpyridine avec la 8-aminoquinoléine :

0,5 g de 2-méthoxy-6-acetylpyridine (3,3 mmol), 0,47g de 8-aminoquinoléine (3,3 mmol) sont dissous dans 5,6 mL de méthanol fraîchement distillé. 0,07 mL d'HCOOH sont ajoutés et le milieu réactionnel est porté à reflux pendant 4 jours puis évaporé sous vide. L'huile brune obtenue est purifiée par colonne chromatographique flash sur silice (éluant: CH₂Cl₂ 100% puis CH₂Cl₂/AcOEt, 80/20). Un solide pur est obtenu. RMN¹H : δ_{H} (300 MHz, CD₂Cl₂) 1.88 (s, 3H), 3.92 (s, 3H), 3.93(s, 3H), 6.23 (d, 1H, *J* 2.3 Hz), 6.52 (dd, 1H, *J* 8.3 et 0.6 Hz), 6.74 (dd, 1H, 8.2 et 0.6 Hz), 6.95 (d, 1H, *J* 8.6 Hz), 7.07 (m, 3H), 7.35 (dd, 1H, *J* 7.4 et 4.3 Hz), 7.41-7.51 (m, 2H), 7.66 (dd, 1H, *J* 8.3 et 7.3 Hz), 8.01 (dd, 1H, *J* 8.3 et 1.6 Hz), 8.73 (dd, 1H, *J* 4.2 et 1.7 Hz) ppm ;

RMN ¹³C : δ_{C} (75MHz, CD₂Cl₂) 31.0, 53.5, 53.6, 58.6, 108.8, 109.8, 112.5, 113.9 (2C), 116.5, 121.2, 125.4, 128.8, 129.9, 136.0, 136.2, 139.3, 139.5 (2C), 141.0, 147.9, 155.9, 163.8, 164.0, 164.6ppm.

IR : 3384, 2972, 2945, 1630, 1575, 1477, 1461, 1430, 1254, 1053, 797 cm⁻¹.

SM : EI *m*/*z*: 410, 395, 302, 286, 258.

La formule développée du produit obtenu A est la suivante :

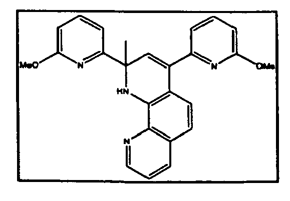

### Exemple 6 :

1,5 g (2,94 mmol) de produit A obtenu selon l'Exemple 3 et 0,21 g (0,18 mmol) de tétrakispalladium (0) sont dissous dans 12 mL de toluène. Une solution dégazée de 1,25 g (11,8 mmol) de Na₂CO₃ dans 6 mL d'eau distillée et 0,86 g (7,0 mmol) d'acide phényl boronique sont ajoutés. Le milieu réactionnel est porté à reflux pendant 24h. Puis 4,5 mL d'une solution aqueuse à 20% de NH₃ diluée dans 30mL d'une solution aqueuse saturée de Na₂CO₃ sont ajoutés. La phase aqueuse est extraite avec CH₂Cl₂ (3x50mL), les phases organiques réunies sont lavées avec de la saumure, séchées sur MgSO₄, filtrées puis évaporées sous vide. Le produit brut est purifié par colonne chromatographique flash (éluant : CH₂Cl₂ 100%). m : 1,56g. Rendement : 44%.

RMN ¹H : δ_{H} (300 MHz, CD₂Cl₂) 2.01 (s, 3H), 6.37 (d, 1H, *J* 2.2Hz), 6.99 (d, 1H, *J* 8.6Hz), 7.22 (bs, 1H), 7.36-7.60(m, 11H), 7.69-7.85 (m, 3H), 8.02-8.12 (m, 5H), 8.8 (dd, 1H, *J* 4.2 et 1.5Hz) ;

RMN ¹³C : δ_{C} (75MHz, CD₂Cl₂) 31.3, 59.3, 114.0, 115.9, 118.4, 118.8, 119.2, 121.8, 122.4, 125.4, 127.3, 128.9. 128.9, 129.0, 129.2, 129.3, 130.3, 136.1, 136.7, 137.8, 137.9, 139.7, 141.2, 148.0, 156.5, 156.7, 158.1, 166.5 ppm.

IR : 3360, 3058, 2964, 1633, 1587, 1564, 1508, 1468, 1441, 1379, 1260, 1158, 1092, 1061, 1026, 989, 814, 800, 760, 692, 659 cm⁻¹.

SM : EI *m*/*z*: 502, 487 , 348, 243.

La formule développée du produit obtenu A est la suivante :

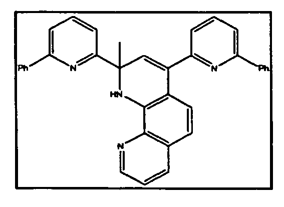

### Exemple 7 :

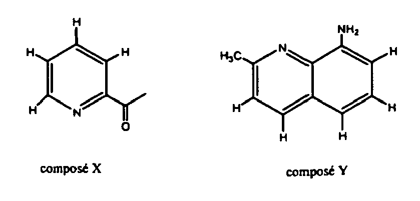

### Synthèse de la 2-méthyl-8-aminoquinoléine (composé Y) Ziessel, R.; Weibel, N.; Charbonnière, L. S. Synthesis 2006, 18, 3128-3133 :

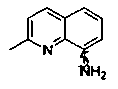

2,110 g de 8-nitroquinaldine (11,19 mmol) sont dissous dans 34 mL de HI (57% aq). Le mélange réactionnel est chauffé à 90°C pendant 2h. A température ambiante, le milieu réactionnel est neutralisé avec 150 mL d'une solution aqueuse saturée de NaHCO₃. La phase aqueuse est extraite avec AcOEt (3x50 mL). Les phases organiques réunies sont lavées avec une solution aqueuse saturée de Na₂S₂O₃ (2x50 mL) puis par de la saumure (2x50 mL). Les phases organiques sont séchées MgSO₄, filtrées puis évaporées sous vide. Le produit brut est purifié par colonne chromatographique flash sur silice (éluant : CH₂Cl₂ 100%). 1,251 g de solide beige sont obtenus. Rendement : 89%.

δ_{H} (300 MHz, CD₂Cl₂) 2.69 (s, 3H), 4.96 (bs, 2H, NH₂), 6.88 (dd, 1H, *J* 7.4 et 1.3 Hz), 7.09 (dd, 1H, *J* 8.1 et 1.3 Hz), 7.25 (m, 2H), 7.96 (d, 1H, *J* 8.4 Hz); RMN ¹³C :

δ_{C} (75MHz, CD₂Cl₂) 25.0, 109.8, 115.6, 122.2, 126.4, 127.0, 136.0, 137.8, 143.7, 156.3 ppm ;

IR : 3467, 3385, 3343, 2365, 3048, 2917, 1616, 1595, 1563, 1507, 1475, 1431, 1373, 1344, 1323, 1284, 1274, 1243, 1137, 1080, 1032, 828, 794, 744, 715, 692 cm⁻¹.

SM : EI *m*/*z*: 158, 131, 103.

### Réaction de la 2-acétylpyridine avec la 2-méthyl-8-aminoquinoléine :

1 g de 2-méthyl-8-aminoquinoline (6,32 mmol), 1,42 mL de 2-acétylpyridine et quelques gouttes d'HCOOH sont dissous dans 10mL de MeOH fraîchement distillé. Le mélange réactionnel est agité pendant 5 jours à reflux. Le milieu réactionnel est ensuite évaporé sous vide. Le produit brut est ensuite purifié par colonne chromatographique flash sur silice (éluant : CH₂Cl₂/AcOEt, 90/10 puis AcOEt 100%). 0,823 mg d'un solide jaune marron sont obtenus, soit un rendement de 43%.

RMN ¹H : δ_{H} (300 MHz, CD₂Cl₂) 1.89 (s, 3H), 2.72 (s, 3H), 6.12 (d, 1H, *J* 2.3 Hz), 6.89 (d, 1H, *J* 9.3 Hz), 6.93 (bs, 1H), 7.12 (ddd, 1H, *J* 7.2, 4.7 et 1.5 Hz), 7.23 (t, 2H, *J* 7.2 Hz), 7.28 (ddd, 1H, *J* 7.6, 4.8 et 1.2 Hz), 7.47 (dt, 1H, *J* 7.8 et 1.1 Hz), 7.56-7.66 (m, 2H), 8.75 (td, 1H, *J* 7.7 et 1.9 Hz), 7.90 (d, 1H, *J* 8.4 Hz), 8.60 (ddd, 1H, *J* 4.7, 1.7 et 0.9 Hz), 8.67 (ddd, 1H, *J* 4.9, 1.9 et 1.0 Hz) ppm ;

RMN ¹³C: δ_{C} (75MHz, CD₂Cl₂) 25.9, 30.8, 59.1, 113.8, 115.5, 120.4, 121.9, 122.6, 122.7, 123.9, 124.1, 136.2, 136.5, 136.8, 136.9, 137.0, 140.0, 149.6 (2C), 156.9, 158.4, 166.8 ppm ;

IR : 3373, 3051, 2964, 2921, 1633, 1602, 1584, 1564, 1552, 1516, 1646, 1429, 1385, 1369, 1259, 1090, 1044, 1019, 993, 835, 785, 746, 706, 687 cm⁻¹.

SM : EI *m*/*z*: 364, 349 , 286.

La formule développée du produit obtenu A est la suivante :

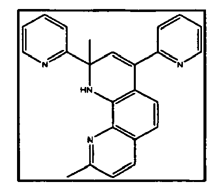

### Exemple 8 :

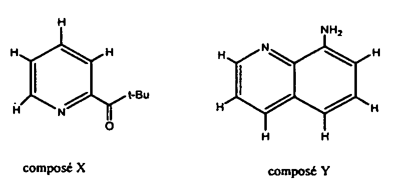

Dans un ballon surmonté d'un Dean-Stark modifié pour récupérer le solvant le plus lourd dont le réservoir est rempli de tamis moléculaire 3 Å, on met 0,85 g (5,2 mmol) de 2,2-dimethyl-1-pyridin-2-ylpropan-1-one, . 1,12 g (7,77 mmol) de 8-aminoquinoléine, 15 mL de toluène sec et 76 mg (0,4 mmol) d'acide p-toluène sulfonique. Le mélange réactionnel est agité pendant 3 jours à 150°C. Le solvant est ensuite évaporé en tirant sous vide. La 8-aminoquinoléine est éliminée par colonne chromatographique sur alumine (éluant : CH₂Cl₂/AcOEt, 80/20). Le produit cristallise dans la cétone de départ. Les cristaux sont lavés au n-pentane puis tirés sous vide pour sécher le produit. 0,53g d'un solide presque translucide sont obtenus (rendement : 35%).

RMN ¹H : δ_{H} (300 MHz, CD₂Cl₂) 1.41 (s, 9H), 6.70 (dd, 1H, *J* 7.3 et 1.45 Hz), 6.81 (dt, 1H, *J* 7.8 et 1.15Hz), 6.93 (ddd, 1H, *J* 7.9, 5.0 et 1.2Hz), 7.12-7.24 (m, 2H), 7.28-7.40 (m, 2H), 7.27 (dm, 1H, *J* 7.3Hz), 8.02 (dd, 1H, *J* 8.4 et 1.74 Hz), 8.41 (ddd, 1H, *J* 5.0, 1.8 et 1.2 Hz) ppm ; RMN ¹³C : δ_{C} (75MHz, CD₂Cl₂) 28.7, 40.7, 118.3, 121.4, 122.4, 122.5, 126.5, 128.9, 135.3, 135.9, 141.4, 148.5, 149.3, 149.6, 157.2, 178.7 ppm ;

IR : 3037, 2929, 1644, 15814, 1560, 1496, 1477, 1462, 1426, 1391, 1363, 1311, 1251, 1217, 1079, 1056, 1033, 1002, 986, 830, 809, 791, 756, 721 cm⁻¹.

GC/MS : 232, 205, 155, 128, 101, 78, 57

La formule développée du produit obtenu B est la suivante :

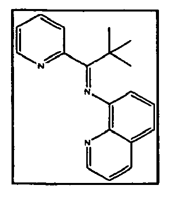

### Exemple 9 :

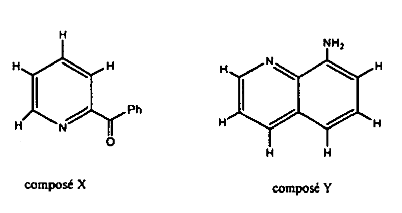

Dans un ballon surmonté d'un Dean-Stark modifié pour récupérer le solvant le plus lourd dont le réservoir est rempli de tamis moléculaire 3Å, on met 1,48 g (8,08 mmol) de 2-benzoylpyridine, 1,17 g (8,11 mmol) de 8-aminoquinoléine, 25 mL de toluène sec et 122,3 mg (0,64 mmol) d'acide p-toluène sulfonique. Le mélange réactionnel est agité pendant 3 jours à 150°C. Le solvant est ensuite évaporé en tirant sous vide. La 8-aminoquinoléine est éliminée par colonne chromatographique sur alumine (éluant : CH₂Cl₂/AcOEt, 80/20). Le produit obtenu est solubilisé dans 30 mL de méthanol et 120 mL de n-heptane sont rajoutés. Les solvants sont évaporés par distillation azéotropique éliminer le méthanol et faire précipiter le produit désiré.

1,3 g de solide jaune sont obtenus (rendement : 52%)

IR : 3053, 1641, 1582, 1563, 1494, 1466, 1429, 1379, 1367, 1311, 1244, 1073, 1054,1027, 993, 958, 829, 792, 764, 754, 740, 723, 713, 638, 616, 599, 583 cm⁻¹.

GC/MS : 309, 231, 205, 181, 154, 128, 101, 77, 51

La formule développée du produit obtenu B est la suivante :

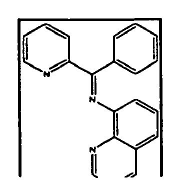

## Revendications

1. Composé organique azoté utilisable comme ligand précurseur en catalyse homogène **caractérisé en ce qu'**il répond à la formule générale : 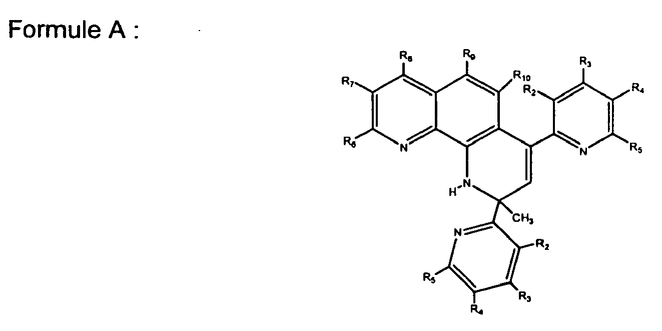 dans laquelle R₂ à R₁₀, identiques ou différents, sont choisis parmi l'hydrogène, des groupements alkyles, saturés ou non saturés, cycloalkyles ou aromatiques, aryles ou aralkyles, éventuellement substitués, des groupements alcoxy, aryloxy ou amino, ou les halogénures.

2. Composé selon la revendication précédente **caractérisé en ce que** les groupements R₂ à R₁₀, identiques ou différents, représentent des radicaux organiques dans lesquels un ou plusieurs atomes d'hydrogène sont remplacés par des halogénures, par exemple un fluorure, ou des groupements comportant au moins un hétéroatome tel que un oxygène, un azote, un soufre, un phosphore ou un silicium, lesdits hétéroéléments pouvant être contenus dans des cycles alkyles saturés ou insaturés ou aromatiques.

3. Composé organique azoté utilisable comme ligand précurseur en catalyse homogène **caractérisé en ce qu'**il répond à la formule générale : 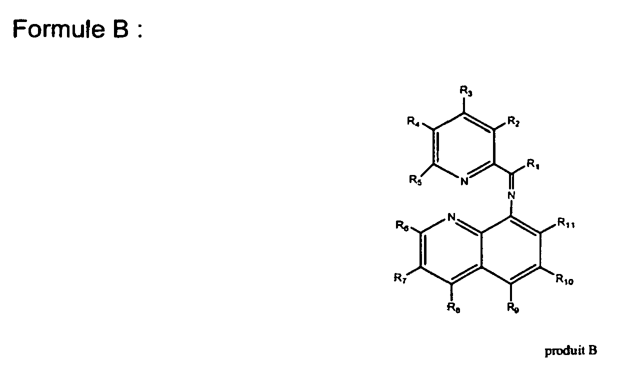 dans laquelle R₁ à R₁₁, identiques ou différents, sont choisis parmi l'hydrogène, des groupements alkyles, saturés ou non saturés, cycloalkyles ou aromatiques, aryles ou aralkyles, éventuellement substitués, des groupements alcoxy, aryloxy ou amino, ou les halogénures, R₁ étant différent du radical méthyle et de l'hydrogène.

4. Composé selon la revendication 3 **caractérisé en ce que** les groupements R₁ à R₁₁, identiques ou différents, représentent des radicaux organiques dans lesquels un ou plusieurs atomes d'hydrogène sont remplacés par des halogénures, par exemple un fluorure, ou des groupements comportant au moins un hétéroatome tel que un oxygène, un azote, un soufre, un phosphore ou un silicium, lesdits hétéroéléments pouvant être contenus dans des cycles alkyles saturés ou insaturés ou aromatiques.

5. Procédé de fabrication des produits A et B selon l'une des revendications 1 à 4 comprenant au moins un étape dans laquelle on fait réagir, de préférence dans un solvant, un composé X appartenant à la famille des pyridines substituées comportant au moins une fonction cétone, avec un composé Y appartenant à la famille des amino-quinoléines et de leurs dérivés, ledit composé X répondant à la formule générale suivante : 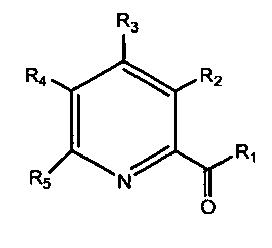 et ledit composé Y répondant à la formule générale 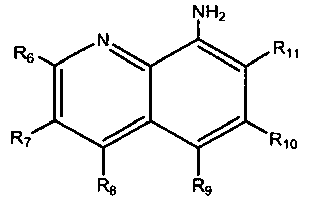 avec R₁ à R_{11,} identiques ou différents, sont choisis parmi l'hydrogène, des groupements alkyles, saturés ou non saturés, cycloalkyles ou aromatiques, aryles ou aralkyles, éventuellement substitués, des groupements alcoxy, aryloxy ou amino, ou les halogénures, R₁ étant différent de l'hydrogène.

6. Procédé selon la revendication 5 dans lequel la température comprise de préférence entre 20 et 250°C.

7. Procédé selon l'une des revendications 5 ou 6 dans lequel la réaction entre le composé X et le composé Y se fait dans un solvant choisi parmi les solvants organiques classiques polaires ou apolaires, protiques ou aprotiques, tels que les hydrocarbures aromatiques ou aliphatiques tels que le toluène, le xylène, le cyclohexane, les solvants chlorés tels que le dichlorométhane, les solvants nitrés tel que l'acétonitrile, les alcools tels que le méthanol ou l'éthanol, seuls ou en mélange.

8. Procédé selon l'une des revendications 1 à 7 dans lequel la réaction entre le composé X et le composé Y est réalisée en présence d'un catalyseur choisi parmi les acides de Bronsted ou les acides de Lewis.

9. Procédé selon l'une des revendications 1 à 8 dans lequel l'eau libérée pendant la réaction entre les composés X et Y est piégée durant la réaction en ajoutant un desséchant tel que du tamis moléculaire ou éliminée par distillation azéotropique avec le solvant de la réaction.

10. Procédé selon l'une des revendications 1 à 9 dans lequel le rapport molaire entre le composé X et le composé Y est compris entre 10 et 0,1, de préférence entre 5 et 0,2.

11. Procédé selon l'une des revendications 1 à 10 dans lequel le produit principal obtenu lors de la réaction de X avec Y est isolé et purifié par précipitation, cristallisation ou séparation par chromatographie en phase liquide sur une colonne d'alumine ou de silice.

## Claims

1. A nitrogen-containing organic compound usable as a precursor ligand in homogeneous catalysis, **characterized in that** it meets the general formula as follows: 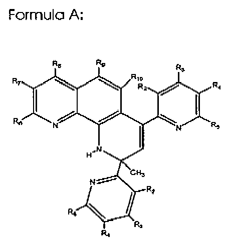 wherein R₂ to R₁₀, Identical or different, are selected from among hydrogen, alkyl groups, saturated or not, cycloalkyl or aromatic, aryl or aralkyl, possibly substituted, alkoxy, aryloxy or amino groups, or hologenides.

2. A compound as claimed in the previous claim, **characterized in that** groups R₂ to R₁₀, identical or different, represent organic radicals wherein one or more hydrogen atoms are replaced by halogenides, a fluoride for example, or groups comprising at least one heteroatom such as oxygen, nitrogen, sulfur, phosphorus or silicon, and said heteroelements can be contained in saturated or unsaturated or aromatic alkyl rings.

3. A nitrogen-contoining organic compound usable as a precursor ligand in homogeneous catalysis, **characterized in that** it meets the general formula as follows: 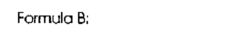 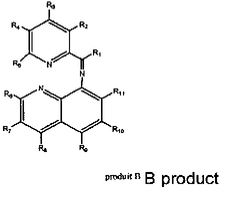 wherein R₁ to R₁₁, identical or different, are selected from among hydrogen, alkyl groups, saturated or not, cycloalkyl or aromatic, aryl or aralkyl, possibly substituted, alkoxy, aryloxy or amino groups, or hologenides. R₁ being different from the methyl radical and the hydrogen.

4. A compound as claimed in claim 3. **characterized in that** groups R₁ to R₁₁, identical or different, represent organic radicals wherein one or more hydrogen atoms are replaced by hologenides, a fluoride for example, or groups comprising at least one heteroatom such as oxygen, nitrogen, sulfur, phosphorus or silicon, and said heteroelements can be contained in saturated or unsaturated or aromatic alkyl rings.

5. A method of producing products A and B as claimed in any one of claims 1 to 4, comprising at least one stage of reacting, preferably In a solvent, a compound X belonging to the substituted pyridine family comprising at least one ketone function with a compound Y belonging to the aminoquinoleine family and their derivatives, said compound X meeting the general formula as follows: 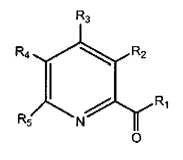 and said compound Y meeting the general formula Is as follows: 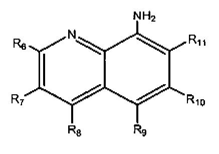 wherein R₁ to R₁₁, identical or different, are selected from among hydrogen, alkyl groups, saturated or not, cycloalkyl or aromatic, aryl or aralkyl, possibly substituted, alkoxy, aryloxy or amino groups, or halogenides, R₁ being different from hydrogen.

6. A method as claimed in claim 5, wherein the temperature preferably ranges between 20°C and 250°C.

7. A method as claimed in any one of claims 5 or 6, wherein the reaction between compound X and compound Y Is carried out in a solvent selected from among conventional organic solvents, polar or apolar, protic or aprotic, such as aromatic or aliphatic hydrocarbons like toluene, xylene, cyclohexane, chlorinated solvents such as dichloromethane, nitro solvents such as acetonitrile, alcohols such as methanol or ethanol, alone or in admixture,

8. A method as claimed in any one of claims 1 to 7, wherein the reaction between compound X and compound Y is preferably carried out in the presence of a catalyst selected from among Bronsted acids or Lewis acids.

9. A method as claimed in any one of claims 1 to 8, wherein the water released upon reaction between compounds X and Y is trapped during the reaction by adding a desiccant such as molecular sieve or eliminated through azeotropic distillation with the reaction solvent.

10. A method as claimed in any one of claims 1 to 9, wherein the molar ratio between compound X and compound Y ranges between 10 and 0.1, preferably between 5 and 0.2.

11. A method as claimed in ony one of claims 1 to 10, wherein the main product obtained during the reaction of X with Y is isolated and purified by precipitation, crystallization or liquid chromatography separation on an alumina or silica column.

## Patentansprüche

1. Stickstoffhaltige organische Verbindung, die als Vorläufer eines Liganden für die homogene Katalyse verwendbar ist, **dadurch gekennzeichnet, dass** sie der folgenden allgemeinen Formel entspricht: 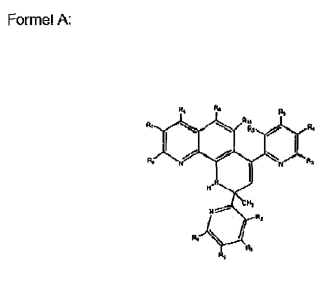 wobei R₂ bis R₁₀, gleich oder verschieden, ausgewählt sind aus Wasserstoff, gesättigten oder ungesättigten Alkylgruppen, Cycloalkylgruppen oder aromatischen Gruppen, Aryl- oder Aralkylgruppen, gegebenenfalls substituiert, Alcoxy-, Aryloxy- oder Aminogruppen oder Halogeniden.

2. Verbindung nach dem vorhergehenden Anspruch, **dadurch gekennzeichnet, dass** die Gruppen R₂ bis R₁₀, gleich oder verschieden, für organische Reste stehen, bei denen ein oder mehrere Wasserstoffatome durch Halogenide, zum Beispiel ein Fluorid, oder Gruppen ersetzt sind, die mindestens ein heteroatom, wie etwa ein Saueretoff-, ein Stickstoff-, ein Schwefel-, ein Phosphor- oder ein Siliciumatom umfassen, wobei die Heteroelemente in gesättigten oder ungesättigten oder aromatischen Ringen enthalten sein können.

3. Stickstoffhaltige organische Verbindung, die als Vorläufer eines Liganden für die homogene Katalyse verwendbar ist, **dadurch gekennzeichnet, dass** sie der folgenden allgemeinen Formel entspricht: 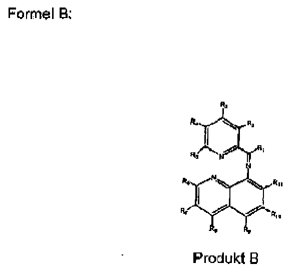 wobei R₁ bis R₁₁, gleich oder verschieden, ausgewählt sind aus Wasserstoff, gesättigten oder ungesättigten Alkylgruppen, Cycloalkylgruppen oder aromatischen Gruppen, Aryl- oder Aralkylgruppen, gegebenenfalls substituiert, Alcoxy-, Aryloxy- oder Aminogruppen oder Halogeniden, wobei R₁ verschieden von einem Methylrest oder Wasserstoff ist.

4. Verbindung nach Anspruch 3, **dadurch gekennzeichnet, dass** die Gruppen R₁ bis R₁₁, gleich oder verschieden, für organische Reste stehen, bei denen ein oder mehrere Wasserstoffatome durch Halogenids, zum Beispiel ein Fluorid, oder Gruppen ersetzt sind, die mindestens ein Heteroatom, wie etwa ein Sauerstoff-, ein Stickstoff-, ein Schwefel-, ein Phosphor- oder ein Siliciumatom umfassen, wobei die Heteroelemente in gesättigten oder ungesättigten oder aromatischen Ringen enthalten sein können.

5. Verfahren zur Herstellung der Produkte A und B nach einem der Ansprüche 1 oder 4, das mindestens einen Schritt umfasst, bei dem, bevorzugt in einem Lösemittel, eine Verbindung X, die zur Familie der substituierten Pyridine gehört, die mindestens eine Ketonfunktion umfassen, mit einer Verbindung Y umgesetzt wird, die zur Familie der Aminochinoleine gehört und deren Derivaten, wobei die Verbindung X der folgenden allgemeinen Formal entspricht: 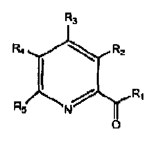 und die Verbindung Y der folgenden allgemeinen Formel entspricht: 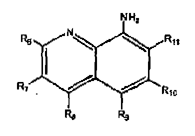 worin R₁ bis R₁₁, gleich oder verschieden, ausgewählt sind aus Wasserstoff, gesättigten oder ungesättigten Alkylgruppen, Cycloalkylgruppen oder aromatischen Gruppen, Aryl- oder Aralkylgruppen, gegebenenfalls substituiert, Alcoxy-, Aryloxy- oder Amingruppen oder Halogeniden, wobei R₁ verschieden von einem Methylrest oder Wasserstoff ist.

6. Verfahren nach Anspruch 5, wobei die Temperatur bevorzugt im Bereich zwischen 20 und 250 °C liegt.

7. Verfahren nach einem der Ansprüche 5 oder 6, wobei die Reaktion zwischen der Verbindung X und der Verbindung Y in einem Lösemittel erfolgt, das aus den herkömmlichen protischen oder aprotischen, polaren oder apolaren organischen Lösemitteln ausgewählt ist, wie etwa den aromatischen oder aliphatischen Kohlenwasserstoffen, wie etwa Toluen, Xylen, Cyclohexan, den.chlorierten Lösemitteln, wie etwa Dichlormethan, den nitrierten Lösemitteln, wie etwa Acetonitril, den Alkoholen, wie etwa Methanol oder Methanol, allein oder als Gemisch.

8. Verfahren nach einem der Ansprüche 1 bis 7, wobei die Reaktion zwischen der Verbindung X und der Verbindung Y in Gegenwart eines Katalysators ausgeführt wird, der aus den Brönsted-Säuren oder den Lewis-Säuren ausgewählt ist.

9. Verfahren nach einem der Ansprüche 1 bis 6, wobei das während der Reaktion zwischen den Verbindungen X und Y freigesetzte Wasser, während der Reaktion gebunden wird, indem ein Trockenmittel, wie etwa ein Molekularsieb, zugefügt wird, oder durch azeotrope Destillation mit dem Lösemittel der Reaktion entfernt wird.

10. Verfahren nach einem der Ansprüche 1 bis 9, wobei das Molverhältnis zwischen der Verbindung X und der Verbindung Y im Bereich zwischen 10 und 0,1, bevorzugt zwischen 5 und 0,2 liegt.

11. Verfahren nach einem der Ansprüche 1 bis 10, wobei das Hauptprodukt, das während der Reaktion von X mit Y erhalten wird, durch Ausfällen, Kristallisieren oder Trennen mittels Flüssigphasenctrromatographie auf einer Aluminium- oder Silioiumidoxidsäule isoliert und gereinigt wird.
